# EUROPÄISCHE PATENTANMELDUNG

(11) **EP 1 364 670 A2**
(43) Veröffentlichungstag der Anmeldung: **26.11.2003**
(21) Anmeldenummer: 03011082.9
(22) Anmeldetag: 16.05.2003
(51) Int. Cl.: A61M 5/34

(54) **Einmalspritze, sowie Verfahren zur Herstellung einer derartigen Spritze**

(30) Priorität: 25.05.2002 DE 10223421
(71) Anmelder: Schott Glas, 55122 Mainz (DE)
(72) Erfinder: Fabian, Arthur, 9000 St. Gallen (CH); Koller, Horst, 9032 Engelburg (CH); Holschumacher, Ralf, Dr., 9032 Engelburg (CH); Eichhorn, Bertram, 9032 Engelburg (CH); Kadur, Thomas, Boyle, Mississippi 38730 (US)
(74) Vertreter: Fuchs Mehler Weiss & Fritzsche

(57) **Zusammenfassung**

Es werden ein Verfahren zur Herstellung einer Spritze sowie eine Spritze, insbesondere eine Einmalspritze, mit einem Spritzenkörper, der einen zylindrischen Vorratsraum für eine zu injizierende Flüssigkeit, einen Kolbenstopfen und eine damit verbundene, axial verschiebbare Kolbenstange umfasst, beschrieben, wobei dem Spritzenkörper endseitig eine Fingerauflage sowie ein hierzu entgegengesetzter Endabschnitt zur Halterung einer Kanüle in einem Nadelkanal zugeordnet ist. Die Kanüle ist hierbei in dem Nadelkanal eingeklemmt.

## Beschreibung

Die vorliegende Erfindung betrifft eine Spritze, insbesondere eine Einmalspritze, mit den Merkmalen des Oberbegriffs des Patentanspruchs 1 sowie ein Verfahren zur Herstellung eines entsprechenden Spritzenkörpers mit den Merkmalen des Oberbegriffs des Patentanspruchs 9.

Spritzen, insbesondere Einmalspritzen, sind in vielfacher Ausgestaltung bekannt und werden vorzugsweise auch als vorgefüllte Einmalspritzen, die einen Glasspritzenkörper und insbesondere einen Kunststoffspritzenkörper aufweisen, verwendet. Hierbei besitzen diese bekannten Einmalspritzen einen Spritzenkörper, der einen zylindrischen Vorratsraum für eine zu injizierende Flüssigkeit, einen Kolbenstopfen und eine damit verbundene und axial verschiebbare Kolbenstange umfasst, wobei dem Spritzenkörper endseitig eine Fingerauflage sowie einen hierzu entgegengesetzten Endabschnitt zur Halterung einer Nadel, die auch üblicherweise auch als Kanüle bezeichnet wird, zugeordnet ist. Hierbei ist bei der bekannten Spritze die Kanüle in einem Nadelkanal angeordnet, wobei die Kanüle durch Verkleben am Endabschnitt der Spritze und innerhalb des Nadelkanals fixiert ist.

Die bekannten Spritzen weisen jedoch den Nachteil auf, dass hierbei aufgrund der Verklebung der Kanüle mit dem Endabschnitt der hierfür verwendete Kleber in Kontakt mit der zu injizierenden Flüssigkeit kommen kann, was zur Folge hat, dass der jeweils verwendete Kleber zugelassen werden muss und somit gewisse gesetzliche Auflagen zu erfüllen hat, was einerseits die Auswahl der möglichen Kleber einengt und andererseits einen erheblichen Aufwand vor und bei der Herstellung der Spritzen verursacht.

Die DE 29 39 180 A1 offenbart eine Ampullen-Einweg-Injektionsspritze, bei der der das Medikament enthaltene Vorratsbehälter und das die Nadel aufnehmende Ansatzstück separat hergestellt und anschließend montiert wird. Die Nadel ist hierbei im Ansatzstück eingeklebt.

Die DE 44 38 360 A1 offenbart eine vorfüllbare Einmalspritze mit einem Füllvolumen < als 5 ml. Der Spritzenkörper mit Spritzenzylinder, Griffleiste und Spritzenkopf ist aus glasig transparentem Kunststoff mit sehr kleiner Gasdurchlässigkeit und gegen Gammastrahlen und/oder Ethylenoxydgas resistent ausgeführt, wobei die Injektionsnadel unmittelbar in den Kunststoffspritzenkopf integriert ist. Diese Integrierung kann dergestalt erfolgen, dass der Bereich des Spritzenkopfes, in dem die Injektionsnadel gehaltert ist, als Umspritzung der Nadel ausgeführt ist.

Der vorliegenden Erfindung liegt die Aufgabe zugrunde, eine Spritze der vorstehend angegebenen Art zur Verfügung zu stellen, die eine besonders einfache Bauweise besitzt.

Diese Aufgabe wird erfindungsgemäß durch eine Spritze mit den kennzeichnenden Merkmalen des Patentanspruchs 1 gelöst.

Die erfindungsgemäße Spritze, insbesondere die erfindungsgemäße Einmalspritze, weist einen Spritzenkörper auf, der einen zylindrischen Vorratsraum für eine zu injizierende Flüssigkeit, einen Kolbenstopfen und eine damit verbundene und axial verschiebbare Kolbenstange umfasst. Des weiteren ist bei der erfindungsgemäßen Spritze der Spritzenkörper endseitig mit einer Fingerauflage sowie mit einem hierzu entgegengesetzten Endabschnitt zur Halterung einer Kanüle in einem Nadelkanal zugeordnet, wobei im Unterschied zum vorstehend genannten Stand der Technik bei der erfindungsgemäßen Spritze die Kanüle nicht mit dem Endabschnitt verklebt sondern statt dessen die Kanüle in dem Nadelkanal ohne Verwendung eines entsprechenden Klebstoffes eingeklemmt ist. Mit anderen Worten verzichtet somit die erfindungsgemäße Spritze, die vorzugsweise auch als Einmalspritze konfiguriert ist, auf den Klebstoff, der bei der herkömmlichen Spritze zur Fixierung der Kanüle erforderlich ist, und fixiert die Kanüle am und insbesondere im Endabschnitt über einen Klemmeingriff zwischen den entsprechenden Teilen des Nadelkanals und der äußeren Kanülenwandung.

Die erfindungsgemäße Spritze weist eine Reihe von Vorteilen auf. So ist zunächst festzuhalten, dass aufgrund des Einklemmens der Kanüle in dem Nadelkanal und des Verzichts auf jeglichen Klebestoff die Herstellung der erfindungsgemäßen Spritze erheblich vereinfacht ist, da hier zusätzliche Arbeitsschritte, so beispielsweise die Ausformung eines entsprechenden Bereiches zur Aufnahme des Klebstoffes im Endabschnitt und dessen Anordnung entfallen können. Auch werden bei der erfindungsgemäßen Spritze keine Klebstoff-Härtungszeiten benötigt, was insbesondere somit die Herstellung der erfindungsgemäßen Spritze beschleunigt. Darüber hinaus verzichtet die erfindungsgemäße Spritze vollständig auf den bei der herkömmlichen Spritze erforderlichen Klebstoff, so dass dementsprechend hier keine gesetzlichen Auflagen zu berücksichtigen sind und folglich die erfindungsgemäße Spritze universell einsetzbar ist.

Um eine besonders vielfach einzusetzende Spritze, insbesondere auch eine Einmalspritze, zur Verfügung zu stellen, sieht eine erste Ausführungsform der erfindungsgemäßen Spritze vor, dass hierbei die Kanüle in dem Nadelkanal so fest eingeklemmt ist, dass sie nur mit einer Kraft größer als 30 N und vorzugsweise mit einer Kraft zwischen 60 N und 120 N aus dem Nadelkanal herausziehbar ist. Hierbei wird bei dieser Ausführungsform der erfindungsgemäßen Spritze sichergestellt, dass nach Anwendung der Spritze beim Patient und Injizierung der jeweiligen Flüssigkeit während des Herausziehens der Kanüle sich die Kanüle nicht in unerwünschter Weise vom Spritzenkörper löst und somit auch nicht im Körper des Patienten verbleiben kann.

Um die Größe des Klemmeingriffes der Kanüle im Nadelkanal zu variieren, befindet sich bei einer weiteren Ausführungsform der erfindungsgemäßen Spritze ein Endbereich der Kanüle im Klemmeingriff mit einem zum Spritzenkörper weisenden Bereich des Nadelkanals. Mit anderen Worten ist hierbei die Kanüle nur mit einem begrenzten Ausmaß ihrer axialen Länge im Nadelkanal eingeklemmt, so dass dementsprechend bei dieser Ausführungsform der erfindungsgemäßen Spritze der Nadelkanal vom Spritzenkörper aus gesehen zunächst zylindrisch verläuft, um hier die erforderlichen Klemmeingriffe mit der Kanüle sicherzustellen, wobei sich an diesen zylindrischen Bereich des Nadelkanals ein vorzugsweise konisch aufgeweiteter Bereich anschließt, der die Einführung der Kanüle in den Nadelkanal und insbesondere in dessen zylindrischen Bereich erleichtert.

Bei einer anderen Ausgestaltung der erfindungsgemäßen Spritze klemmt der Nadelkanal über seine gesamte axiale Länge die Kanüle ein, so dass hierbei die Kanüle über die gesamte axiale Ausdehnung des Endabschnittes besonders sicher gehaltert ist.

Um den zuvor beschriebenen Klemmeingriff zwischen der Kanüle und dem Nadelkanal bei der erfindungsgemäßen Spritze herbeizuführen, bestehen grundsätzlich zwei Möglichkeiten.

So sieht die erste Möglichkeit vor, dass hierbei im Klemmbereich der Innendurchmesser des Nadelkanals an den Außendurchmesser der Kanüle so weit angepasst ist, dass der zuvor beschriebene Klemmeingriff zur Halterung der Kanüle im Nadelkanal herbeiführbar ist, wobei insbesondere, abhängig von der Elastizität und Verformbarkeit des Endabschnittes der Nadelkanalinnendurchmesser um 2 % bis 15 %, vorzugsweise um 4 % bis 8 %, kleiner ist als der Außendurchmesser der Kanüle.

Bei der zweiten Möglichkeit weist der Nadelkanalbereich, in den die Kanüle eingeklemmt ist, einen Innendurchmesser auf, der mindestens dem Außendurchmesser der Kanüle entspricht, so dass hierbei die Kanüle ohne großen Kraftaufwand in diesen Nadelkanalabschnitt einbringbar ist. Vorzugsweise ist jedoch der Nadelkanaldurchmesser um 2 % bis 10 % und insbesondere um 4 % bis 8 % größer als der Außendurchmesser der Kanüle, wodurch die Einführung der Kanüle in den Nadelkanal beschleunigt wird. Anschließend wird bei dieser Ausführungsform der aus einem thermoplastischen Kunststoff bestehende Endabschnitt so weit erwärmt, dass dieser Endabschnitt verformbar ist, so dass durch ein gleichzeitigen Verformen des Endabschnittes der erwünschte Klemmeingriff zwischen der Nadelkanalwandung und der darin angeordneten Nadel herbeiführbar ist.

Eine weitere, zusätzliche Fixierung der Kanüle am Endabschnitt kann bei den zuvor beschriebenen beiden Möglichkeiten noch dadurch erreicht werden, dass die Kanüle so in den Nadelkanal eingebracht wird, dass sie axial für eine relativ kurze Länge in den zylindrischen Spritzenkörper hineinragt. Durch ein anschließendes, vorzugsweise radiales Aufweiten dieses in den Spritzenkörper hineinragenden Endes wird erreicht, dass die Kanüle nur noch unter Zerstörung des Endabschnittes aus dem Nadelkanal herausziehbar ist, so dass bei dieser Ausführungsform der erfindungsgemäßen Spritze die Kanüle nicht nur in dem Nadelkanal eingeklemmt sondern zusätzlich noch durch die zuvor beschriebene Aufweitung gehaltert ist.

Bei einer besonders geeigneten Ausführungsform der erfindungsgemäßen Spritze ist der mit der Kanüle versehene Endabschnitt, bei dem die Kanüle in dem Nadelkanal eingeklemmt ist, nicht einstückig mit der Spritze ausgebildet sondern über ein Verbindungselement mit dem zylindrischen Vorratsraum verbunden, so dass hierbei der mit der Kanüle versehene Endabschnitt separat von dem zylindrischen Vorratsraum für die zu injizierende Flüssigkeit hergestellt wird. Über das am Endabschnitt und/oder dem zylindrischen Vorratsraum vorgesehene Verbindungselement erfolgt dann die Arretierung des mit der Kanüle versehenen Endabschnittes an dem zylindrischen Vorratsraum, wobei diese Ausführungsform der erfindungsgemäßen Spritze den wesentlichen weiteren Vorteil aufweist, dass hierbei die Herstellung noch weiter vereinfacht wird, wie dies nachfolgend noch bei dem erfindungsgemäßen Verfahren erläutert ist.

Grundsätzlich kann bei der zuvor beschriebenen Ausführungsform der erfindungsgemäßen Spritze das Verbindungselement beliebig ausgestaltet sein, sofern sichergestellt ist, dass hierbei der mit der Kanüle versehene Endabschnitt sicher mit dem zylindrischen Vorratsraum verbunden ist. Besonders vorteilhaft ist es, wenn das Verbindungselement als Schraubverbindung ausgebildet ist, derart, dass insbesondere der Endabschnitt mit einem entsprechenden Außengewinde versehen ist, so dass dieser in einen entsprechenden, endseitig am Vorratsraum vorgesehenen und mit einem Innengewinde ausgestatteten Verbindungsbereich einschraubbar ist. Selbstverständlich besteht jedoch auch die Möglichkeit, dass ein endseitig am Vorratsraum versehener Verbindungsbereich mit einem Außengewinde versehen ist, auf das ein mit einem Innengewinde versehener Endabschnitt, der den Nadelkanal und die darin eingeklemmte Kanüle aufweist, aufschraubbar ist.

Um bei den zuvor beschriebenen Ausführungsformen der erfindungsgemäßen Spritze die Kanüle nach außen hin abzudecken, sieht eine andere Ausführungsform der erfindungsgemäßen Spritze vor, dass hierbei der Endabschnitt auf seinem äußeren Umfang mit einem Halterungsabschnitt zur lösbaren Halterung eines Kanülenschutzes versehen ist, wobei vorzugsweise dieser Halterungsabschnitt mit einem entsprechenden Gewinde versehen ist, so dass der Kanülenschutz, der allgemein auch als needle shield bezeichnet wird, aufschraubbar ist.

Die vorliegende Erfindung betrifft des weiteren ein Verfahren zur Herstellung eines Spritzenkörpers für die zuvor beschriebenen Ausführungsformen der erfindungsgemäßen Spritze mit den kennzeichnenden Merkmalen des Patentanspruchs 9.

Das erfindungsgemäßen Verfahren zur Herstellung eines Spritzenkörpers für eine Spritze der zuvor beschriebenen Art sieht vor, dass hierbei in einem ersten Arbeitsschritt der zylindrische Vorratsraum hergestellt wird, dass in einem weiteren, vom ersten Arbeitsschritt verschiedenen Arbeitsschritt der mit der im Nadelkanal eingeklemmten Kanüle versehene Endabschnitt hergestellt wird und dass hiernach in einem abschließenden Arbeitsschritt der zylindrische Vorratsraum mit dem Endabschnitt verbunden wird. Mit anderen Worten umfasst das zuvor beschriebene erfindungsgemäße Verfahren zwei, getrennt voneinander vorgesehene Teilarbeitsschritte, wobei der erste Arbeitsschritt zur Herstellung des zylindrischen Vorratsraumes und der weitere, vom ersten Teilarbeitsschritt unterschiedliche Teilarbeitsschritt zur Herstellung des Endabschnittes dient, wobei dieser Endabschnitt dann die im Nadelkanal eingeklemmte Kanüle aufweist. Erst in einem folgenden, abschließenden Arbeitsschritt wird dann der zylindrische Vorratsraum mit dem die Kanüle aufweisenden Endabschnitt verbunden, so daß ein Spritzenkörper resultiert, der lediglich noch mit dem Kolbenstopfen und eine damit verbundene Kolbenstange sowie ggf. endseitig mit einer Fingerauflage versehen werden muss. Hierbei kann jedoch auch das erfindungsgemäße Verfahren derart durchgeführt werden, dass in dem ersten Teilarbeitsschritt der zylindrische Vorratsraum hergestellt wird, der endseitig bereits die Fingerauflage aufweist.

Soll nach dem zuvor beschriebenen erfindungsgemäßen Verfahren eine Einmalspritze insbesondere aus Kunststoff hergestellt werden, so bietet es sich an, die zuvor beschriebenen Teilarbeitsschritte (erster Arbeitsschritt und weiterer Arbeitsschritt) jeweils nach einem Spritzgießverfahren durchzuführen, derart, dass in einem separaten ersten Arbeitsschritt der Vorratsraum und ein hieran angeordneter und mit dem Vorratsraum einstückig ausgebildeter Gewindeabschnitt durch dieses Spritzgießverfahren hergestellt wird, während in dem weiteren, vom ersten Arbeitsschritt getrennt ablaufenden Arbeitsschritt der Endabschnitt mit der im Nadelkanal eingeklemmten Kanüle ebenfalls durch ein Spritzgießverfahren hergestellt wird.

Der wesentliche Vorteil des erfindungsgemäßen Verfahrens ist insbesondere darin zu sehen, dass es aufgrund der Formteilgeometrien technisch wesentlich einfacher ist, Kanülen in ein Spritzgießwerkzeug für die Herstellung von Endabschnitten einzulegen, wobei die Herstellung des zylindrischen Vorratsraumes selbst dann in dem separaten Teilarbeitsschritt unter Verwendung von relativ einfach geformten Spritzgießwerkzeugen besonders schnell und einfach durchzuführen ist. Hierbei kann dann, falls erwünscht, das entsprechende Spritzgießwerkzeug für die Herstellung des Vorratsraumes noch so ausgestaltet sein, dass gleichzeitig mit der Erstellung des Vorratsraumes endseitig eine Fingerauflage vorgesehen wird.

Der nach dem zuvor beschriebenen erfindungsgemäßen Verfahren hergestellte Spritzenkörper lässt sich dann insbesondere durch Einsatz eines Kolbenstopfens in den zylindrischen Vorratsraum und einer damit verbundenen, axial verschiebbaren Kolbenstange zu einer Einmalspritze weiterverarbeiten, wobei die Kanüle dann zusätzlich noch mit einer lösbaren Kanülenabdeckung (needle shield) versehen wird.

Vorteilhafte Weiterbildungen der erfindungsgemäßen Spritze sowie des zuvor beschriebenen erfindungsgemäßen Verfahrens sind in den Unteransprüchen angegeben.

Die erfindungsgemäße Spritze wird nachfolgend anhand von zwei Ausführungsbeispielen näher erläutert. Es zeigen:
- Figur 1: schematisch eine erste Ausführungsform der erfindungsgemäßen Spritze während ihrer unterschiedlichen Stufen der Herstellung;
- Figur 2: schematisch eine zweite Ausführungsform der erfindungsgemäßen Spritze vor dem Zusammenbau des Vorratsraumes mit dem Endabschnitt.

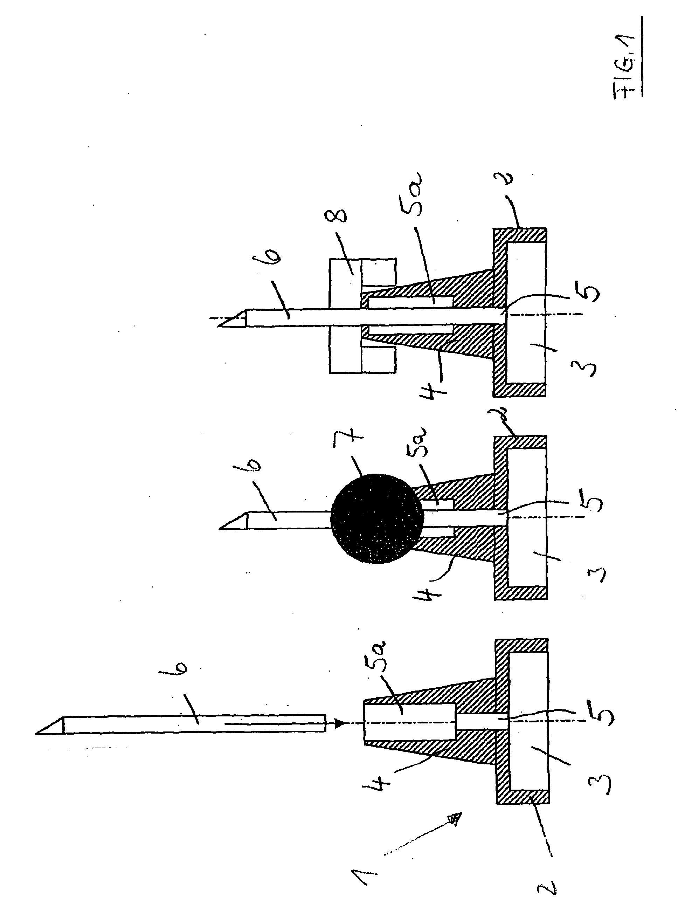

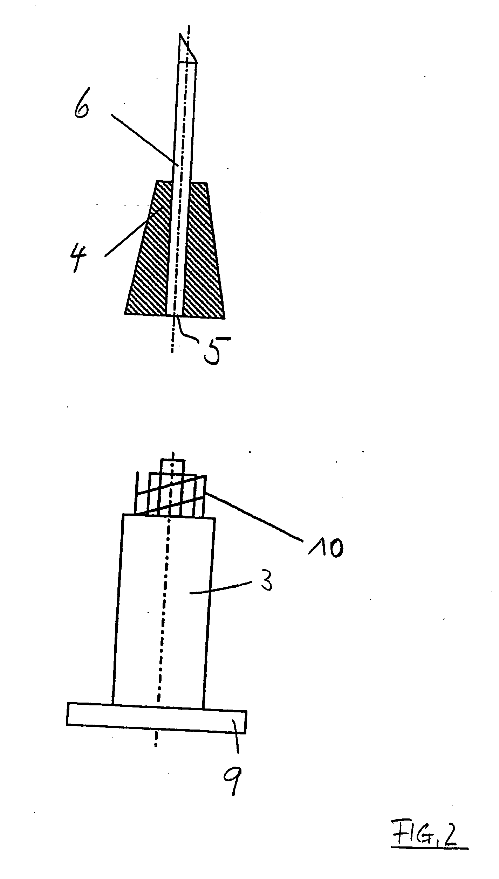

In den Figuren 1 und 2 sind die selben Teile mit den selben Bezugszeichen versehen.

In den drei Abbildungen der Figur 1 ist schematisch eine erste Ausführungsform der insgesamt mit 1 bezeichneten Spritze abgebildet, wobei die drei Teilansichten, die von links nach rechts betrachtet mit erster Teilansicht, zweiter Teilansicht und dritter Teilansicht nachfolgend bezeichnet werden, jeweils die verschiedenen Herstellungsstufen der Spritze 1 wiederspiegeln.

Die in der ersten Teilansicht schematisch abgebildete Spritze 1 weist einen Spritzenkörper 2 auf, wobei der Spritzenkörper 2 einen zylindrischen Vorratsraum 3 zur Aufnahme eines nicht gezeigten Kolbenstopfens und einer damit verbundenen, axial verschiebbaren Kolbenstange (ebenfalls nicht gezeigt) umfasst. Endseitig an dem Spritzenkörper 2 ist eine nicht gezeigte Fingerauflage vorgesehen, mit der die Spritze 1 während ihres Gebrauchs sicher gehaltert werden kann. An dem zur Fingerauflage entgegengesetzten Ende weist die Spritze 1 einen Endabschnitt 4 auf, der mit einem Nadelkanal 5 versehen ist, in den eine Kanüle 6 eingeführt wird, wie dies die erste Teilansicht schematisch abbildet. Hierbei ist der Nadelkanal 5 an seinem ausflußseitigen Ende unter Ausbildung einer zylindrischen Aufweitung 5a erweitert, wobei diese Aufweitung 5a nur bei den herkömmlichen bekannten Spritzen einen Klebstoff aufnimmt, um so die Kanüle 6 am Endabschnitt 4 zu haltern.

Die zylindrische Aufweitung 5a des Nadelkanals 5 ist bei der gezeigten Ausführungsform vergrößert abgebildet, um die Unterschiede im Durchmesser des Nadelkanals 5 und seiner Aufweitung besser zu dokumentieren.

Nach Einsetzen der Kanüle 6 in den Nadelkanal 5, wie dies die zweite Teilansicht der Figur 1 wiedergibt, wird der Endabschnitt 4 im Bereich der Aufweitung 5a erwärmt, wie dies symbolisch in der zweiten Teilansicht durch den dunkleren Kreis 7 angedeutet ist.

Da der Endabschnitt 4 aus einem thermoplastischen Kunststoff gefertigt ist, führt die Erwärmung des Endabschnittes dazu, dass der Endabschnitt mittels eines geeigneten Werkzeuges 8 derart verformbar ist, dass die zylindrische Aufweitung 5a eliminiert wird und dass der Kunststoff des Endabschnittes 4 die in den Nadelkanal 5 eingeführte Kanüle 6 allseitig fest umschließt und somit die Kanüle 6 in den Nadelkanal 5 und insbesondere im Bereich 5a einklemmt.

Die in Figur 1 in drei Teilansichten gezeigte Ausführungsform bildet somit eine Spritze ab, bei der der Endabschnitt 4 einstückig mit dem zylindrischen Vorratsraum 3 ausgebildet ist, während die in Figur 2 gezeigte weitere Ausführungsform eine Spritze 1 darstellt, bei der der zylindrische Vorratsraum 3 über ein als Schraubverbindung ausgestaltetes Verbindungselement 10, das einstückig am zylindrischen Vorratsraum 3 angeordnet ist, mit dem Endabschnitt 4 verbunden wird. Hierbei weist die in Figur 2 gezeigte Ausführungsform, die eine Spritze vor ihrem Zusammenbau darstellt, einen Endabschnitt 4 auf, der mit einem Nadelkanal 5 versehen ist, in den eine Kanüle 6 eingeklemmt ist.

Zur Herstellung dieses Endabschnitts wird vorzugsweise so vorgegangen, dass der Endabschnitt 4 in einem separaten Arbeitsschritt unter Verwendung eines geeigneten Spritzgießwerkzeuges unter Einlegung der Kanüle 6 direkt erstellt wird, wobei der Endabschnitt 4 des weiteren noch ein nicht gezeigtes Innengewinde aufweist, so dass über dieses Innengewinde der Endabschnitt 4 auf die Schraubverbindung 10 des zylindrischen Vorratsraumes 3 aufschraubbar ist. Des weiteren weist der zylindrische Vorratsraum 3 eine mit 9 bezeichnete Fingerauflage auf und wird zur Erstellung der fertigen Spritze mit einem innenliegenden Kolbenstopfen und eine damit verbundene, axial verschiebbare Kolbenstange (beides nicht gezeigt) versehen.

Um die zweiteilige, in Figur 2 gezeigte Ausführungsform der Spritze bzw. des Spritzenrohlings herzustellen, wird in einer ersten Spritzgießmaschine der zylindrische Vorratsraum 3, einschließlich des Verbindungselementes 10 und der Fingerauflage 9, hergestellt, wobei der zylindrische Vorratsraum kopfseitig mit dem Verbindungselement 10 versehen ist, das vorzugsweise aus Luerlock-Adapter ausgebildet ist.

Auf einer zweiten Spritzgießmaschine wird separat von der ersten Spritzgießmaschine der Endabschnitt 4 erstellt, wobei nach Einlegen der Kanüle 6 in die einzelne Kavität des Spritzgießwerkzeuges diese eingelegte Kanüle 6 mit dem thermoplastischen Materials des Endabschnittes 4 umspritzt wird. Gleichzeitig hiermit wird das innerhalb des Endabschnittes 4 vorgesehene Innengewinde (nicht abgebildet) erstellt, so dass nach Entnahme des nunmehr mit der umspritzten Kanüle 6 versehenen Endabschnittes 4 dieses unmittelbar auf das Verbindungselement 10 aufgeschraubt werden kann.

## Patentansprüche

1. Spritze, insbesondere eine Einmalspritze, mit einem Spritzenkörper, der einen zylindrischen Vorratsraum für eine zu injizierende Flüssigkeit, einen Kolbenstopfen und eine damit verbundene, axial verschiebbare Kolbenstange umfasst, wobei dem Spritzenkörper endseitig eine Fingerauflage sowie ein hierzu entgegengesetzter Endabschnitt zur Halterung einer Kanüle in einem Nadelkanal zugeordnet ist, **dadurch gekennzeichnet, dass** die Kanüle (6) in dem Nadelkanal (5) eingeklemmt ist.

2. Spritze nach Anspruch 1, **dadurch gekennzeichnet, dass** die Kanüle (6) in dem Nadelkanal (5) so fest eingeklemmt ist, dass sie nur mit einer Kraft größer als 30 N, vorzugsweise mit einer Kraft zwischen 60 N und 120 N, aus dem Nadelkanal (5) herausziehbar ist.

3. Spritze nach Anspruch 1 oder 2, **dadurch gekennzeichnet, dass** sich ein Endbereich der Kanüle (6) im Klemmeingriff mit einem zum zylindrischen Vorratsraum (2) weisenden Bereich des Nadelkanals (5) befindet.

4. Spritze nach Anspruch 1 oder 2, **dadurch gekennzeichnet, dass** der Nadelkanal (5) über seine gesamte axiale Länge die Kanüle (6) einklemmt.

5. Spritze nach einem der vorangehenden Ansprüche, **dadurch gekennzeichnet, dass** der Endabschnitt (4) aus einem thermoplastischen Kunststoff besteht und dass durch ein Erwärmen des Kunststoffes und einem gleichzeitigen Verformen desselben der Klemmeingriff zwischen dem Nadelkanal (5) und der darin angeordneten Kanüle (6) herbeigeführt ist.

6. Spritze nach einem der vorangehenden Ansprüche, **dadurch gekennzeichnet, dass** der mit der Kanüle (6) versehene Endabschnitt (4) über ein Verbindungselement (10) mit dem zylindrischen Vorratsraum (2) verbunden ist.

7. Spritze nach Anspruch 6, **dadurch gekennzeichnet, dass** das Verbindungselement (10) als Schraubverbindung ausgebildet ist.

8. Spritze nach einem der vorangehenden Ansprüche, **dadurch gekennzeichnet, dass** der Endabschnitt (4) auf seinem äußeren Umfang mit einem Halterungsabschnitt zur lösbaren Halterung eines Kanülenschutzes versehen ist.

9. Verfahren zur Herstellung eines Spritzenkörpers für eine Spritze nach einem der vorangehenden Ansprüche, **dadurch gekennzeichnet, dass** in einem ersten Arbeitsschritt der zylindrischen Vorratsraum hergestellt wird, dass in einem weiteren, vom ersten Arbeitsschritt verschiedenen Arbeitsschritt der mit der eingeklemmten Kanüle versehene Endabschnitt hergestellt wird und dass hiernach in einem abschließenden Arbeitsschritt der zylindrische Vorratsraum mit dem Endabschnitt verbunden wird.

10. Verfahren nach Anspruch 9, **dadurch gekennzeichnet, dass** im ersten Arbeitsschritt der Vorratsraum und ein hieran angeordneter und mit dem Vorratsraum einstückig ausgebildeter Gewindeabschnitt durch ein Spritzgießverfahren hergestellt wird.

11. Verfahren nach Anspruch 9 oder 10, **dadurch gekennzeichnet, dass** in dem weiteren Arbeitsschritt der Endabschnitt mit der im Nadelkanal eingeklemmten Kanüle durch ein Spritzgießverfahren hergestellt wird.

12. Spritzenkörper zur Verwendung in einer Spritze nach einem der Ansprüche 1 bis 8.

13. Spritzenkörper, hergestellt nach einem Verfahren nach Anspruch 9 bis 11.
